(19) Europäisches Patentamt
European Patent Office
Office européen des brevets

(11) Veröffentlichungsnummer: **0 197 375**
**B1**

(12) # EUROPÄISCHE PATENTSCHRIFT

(45) Veröffentlichungstag der Patentschrift: **25.01.89**

(21) Anmeldenummer: **86103685.3**

(22) Anmeldetag: **18.03.86**

(51) Int. Cl.⁴: **C 07 B 55/00,** C 07 C 31/125, C 07 C 35/08, C 07 J 9/00, C 07 C 127/26, C 07 C 67/24

(54) **Verfahren zur Inversion der Konfigurationsekundärer Alkohole.**

(30) Priorität: **28.03.85 DE 3511210**

(43) Veröffentlichungstag der Anmeldung: **15.10.86 Patentblatt 86/42**

(45) Bekanntmachung des Hinweises auf die Patenterteilung: **25.01.89 Patentblatt 89/4**

(84) Benannte Vertragsstaaten: **BE CH DE FR GB IT LI**

(56) Entgegenhaltungen:
**SYNTHESIS, Nr. 8, August 1979, Seiten 561-576, Georg Thieme Publishers; L.J. MATHIAS: "Esterification and alkylation reactions employing isoureas"**
**ROBERT THORNTON MORRISON et al.: "Organic Chemistry", 3. Ausgabe, Seiten 461-463, Allyn and Bacon, Inc., Boston, US.**

(73) Patentinhaber: **BAYER AG, Konzernverwaltung RP Patentabteilung, D-5090 Leverkusen 1 Bayerwerk (DE)**

(72) Erfinder: **Kaulen, Johannes, Dr., Odenthaler Strasse 10, D-5090 Leverkusen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 197 375 B1

LIBER, STOCKHOLM 1989

**Beschreibung**

Die vorliegende Erfindung betrifft ein neues Verfahren zur Inversion der Konfiguration sekundärer Alkohole, bei denen die Hydroxygruppe an ein asymmetrisches Kohlenstoffatom gebunden ist.

Es sind bereits Verfahren zur Inversion der Konfiguration sekundärer Alkohole bekannt. Sie beruhen darauf, daß man sekundäre Alkohole, bei denen die Hydroxyfunktion an ein asymmetrisches Kohlenstoffatom gebunden ist, mit Carbonsäuren in Gegenwart von Azodicarbonsäureester/Triphenylphosphin (Synthesis 1981, 1) oder von Neopentylacetalen des Dimethylformamids (Ann. Chem. 1974, 821) unter Inversion der Konfiguration verestert und die Ester unter Konfigurationserhalt verseift. Bei anderen Verfahren wird der sekundäre Alkohol zunächst in einen Methan- oder p-Toluolsulfonsäureester übergeführt, der dann mit Natrium-, Cäsium- oder Ammoniumsalzen von Carbonsäuren (siehe J. Am. Chem. Soc. 96, (1974) 5876; J. Org. Chem. 46, (1981) 4321; Tetrahedr. Lett. 1972, 3265), mit Kaliumperoxid in Gegenwart von Kronenethern (Tetrahedr. Lett. 1975, 3183) oder mit Kaliumnitrit in Dimethylsulfoxid (Synthesis 1980, 292) invertiert und anschließend zum Alkohol mit invertierter Konfiguration verseift. Diese bekannten Verfahren weisen jedoch erhebliche Nachteile auf: So erfordern sie z. B. den Einsatz kostspieliger Hilfsreagenzien; die Abtrennung der Hilfsreagenzien und/oder die Aufarbeitung der Veresterungsgemische ist schwierig, insbesondere dann, wenn schwer entfernbare Lösungsmittel wie Dimethylsulfoxid oder Dimethylformamid verwendet werden müssen; infolge der Bildung von Nebenprodukten (Olefinen) schwanken die Ausbeuten stark. Die über die Methan- und p-Toluolsulfonsäureester verlaufenden Verfahren haben darüber hinaus den Nachteil, daß sie zwei getrennte Reaktionsschritte erfordern.

Es wurde nun überraschenderweise gefunden, daß bei der Umsetzung von Isoharnstoffethern sekundärer Alkohole, bei denen die Hydroxygruppe an ein asymmetrisches Kohlenstoffatom gebunden ist, mit Carbonsäuren zu Estern dieser sekundären Alkohole Inversion eintritt. Das heißt, die aus den Isoharnstoffethern hergestellten Ester und die aus diesen erhaltenen Alkohole weisen an dem asymmetrischen Kohlenstoffatom die dem Ausgangs-Alkohol entgegengesetzte Konfiguration auf. Da Isoharnstoffether sekundärer Alkohole leicht zugänglich sind, eröffnet das Auffinden, daß die Veresterung der sekundären Alkohole über die Isoharnstoffether unter Inversion der Konfiguration des sekundären Alkohols verläuft, ein neues einfaches wirtschaftliches Verfahren zur Inversion der Konfiguration sekundärer Alkohole.

Die Erfindung betrifft daher ein neues Verfahren zur Inversion der Konfiguration sekundärer Alkohole, bei deren die Hydroxyfunktion an ein asymmetrisches Kohlenstoffatom geknüpft ist, durch Veresterung dieser sekundären Alkohole unter Inversion ihrer Konfiguration und nachfolgendes Verseifen der Ester unter Erhalt der Konfiguration des sekundären Alkohols, das dadurch gekennzeichnet ist, daß man die Veresterung unter Inversion der Konfiguration des sekundären Alkohols in der Weise vornimmt, daß man den sekundären Alkohol durch Addition an ein Carbodiimid zunächst in einen Isoharnstoffether überführt und diesen Isoharnstoffether mit einer Carbonsäure zum Ester der Carbonsäure umsetzt.

Das erfindungsgemäße Verfahren sei anhand des folgenden Reaktionsschemas erläutert:

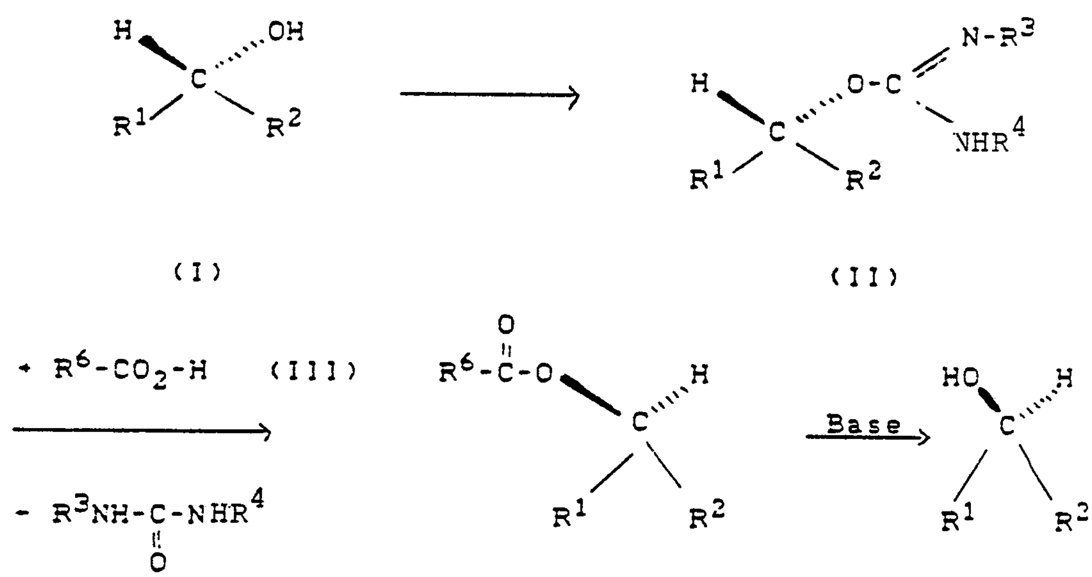

(I) (II) (III) (IV) (V)

Es ist zwar bereits bekannt, daß man Alkohole verestern kann, indem man sie, z. B. durch Addition an ein Carbodiimid, in einen Isoharnstoffether überführt, und diesen Isoharnstoffether mit einer Carbonsäure umsetzt (siehe Synthesis 1979, 561; Angew. Chem. 78, (1966) 483). Über den stereochemischen Verlauf dieser Veresterung bei sekundären Alkoholen ist jedoch bislang nichts bekannt. Es ist als außerordentlich überraschend anzusehen, daß man nach dem erfindungsgemäßen Verfahren die invertierten Ester, und mit

ihnen auch die sekundären Alkohole mit invertierter Konfiguration, nicht nur in hoher Ausbeute, sondern auch außerordentlich hoher optischer Reinheit (bzw. Diastereomeren-Reinheit) erhält, weil zu erwarten war, daß bei der Umsetzung der Isoharnstoffether zu den Carbonsäureestern zumindest teilweise Racemisierung eintritt.

In den sekundären Alkoholen der Formel

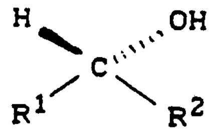

(I)

stehen

$R^1$ und $R^2$ unabhängig voneinander für einen gegebenenfalls substituierten gesättigten oder ungesättigten aliphatischen oder cycloaliphatischen Kohlenwasserstoffrest oder für einen gegebenenfalls substituierten araliphatischen oder aromatischen Kohlenwasserstoffrest oder bilden zusammen einen asymmetrischen Alkylenrest, mit der Maßgabe, daß $R^1/R^2$ ist. Die Natur der Reste $R^1$ und $R^2$ ist ohne Bedeutung für den stereochemischen Verlauf der Veresterungsreaktion; entscheidend ist lediglich daß das zentrale, die OH-Gruppe tragende Kohlenstoffatom ein Asymmetriezentrum ist, d. h. daß die Reste $R^1$ und $R^2$ verschieden sind.

Die Überführung der Alkohole der Formel (I) in die Isoharnstoffether der Formel

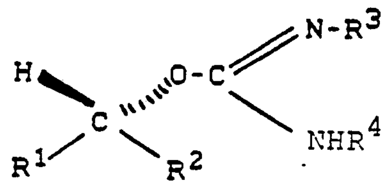

(II)

in der

$R^3$ und $R^4$ unabhängig voneinander für einen gegebenenfalls substituierten Alkyl-, Cycloalkyl-, Aralkyl- oder Arylrest stehen

geschieht durch Addition der sekundären Alkohole der Formel (I) an Carbodiimide der Formel

$R^3\text{-}N=C=N\text{-}R^4$ (VI),

in der

$R^3$ und $R^4$ de unter Formel (II) angegebene Bedeutung haben.

Die Isoharnstoffether der Formel (II) werden durch Umsetzung mit Carbonsäuren der Formel

$R^6\text{-}CO_2H$ (III)

in der

$R^6$ für Wasserstoff oder einen gegebenenfalls substituierten gesättigten oder ungesättigten aliphatischen oder cycloaliphatischen Kohlenwasserstoffrest oder einen gegebenenfalls substituierten araliphatischen oder aromatischen Kohlenwasserstoffrest steht,

unter Inversion der Alkohol-Konfiguration in Ester der Formel

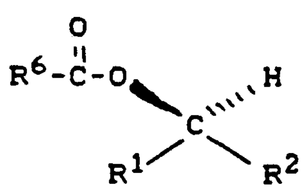

(IV)

überführt, in der

$R^1$, $R^2$ und $R^6$ die unter den Formeln (I) und (III) angegebene Bedeutung besitzen.

Ist das Kohlenstoffatom, an das die Hydroxygruppe gebunden ist, das einzige Asymmetriezentrum des Moleküls, so erhält man nach dem erfindungsgemäßen Verfahren aus der R-Form (S-Form) des sekundären Alkohols der Formel (I) sein S-Enantiomer(R-Enantiomer) in hoher optischer Reinheit.

Besitzt das Molekül dagegen zusätzlich zu dem Kohlenstoffatom, das die Hydroxygruppe trägt, noch ein oder mehrere weitere Asymmetriezentren, so erhält man die Diastereomere dieses sekundären Alkohols der Formel (I) in hoher Diastereomeren-Reinheit.

Für $R^1$, $R^2$ und $R^6$ seien als gesättigte aliphatische Kohlenwasserstoffreste $C_1$-$C_{12}$-Alkylreste wie der Methyl-, Ethyl-, n-Propyl-, iso-Propyl-, n-Butyl-, iso-Butyl-, tert.-Butyl-, n-Pentyl-, 2-Methylpentyl-, n-Hexyl-, i-Hexyl-, 2-Ethyl-hexyl-, und der n-Dodecyl-Rest genannt;

als ungesättigte aliphatische Kohlenwasserstoffreste seien $C_1$-$C_8$-Alkenylreste wie der Allyl-, und der 2-Hexenylrest und Alkinyl-Reste wie der Propargylrest genannt. Als gesättigte cycloaliphatische

Kohlenwasserstoffreste kommen vor allem Cycloalkylreste wie der Cyclopropyl-, Cyclobutyl-, Cyclopentyl-, Cyclohexyl- und der Cycloheptyl-Rest in Betracht. Als ungesättigte cycloaliphatische Kohlenwasserstoffreste seien vor allem der Cyclohexenyl-Rest genannt.

Als aromatische Kohlenwasserstoffreste kommen vor allem der Phenyl- und der Naphthylrest, als araliphatische Kohlenwasserstoffreste Aralkylreste wie der Benzyl-, β-Phenylethyl-, γ-Phenylpropyl- und ω-Phenyl-butylrest in Betracht. Als asymmetrische Alkylenreste, die $R^1$ und $R^2$ zusammen bilden können, seien vor allem substituierte Ethylenreste wie der Propylen-1,2-Rest, ferner der Butylen-1,3- und asymmetrisch substituierte Pentylen-1,5- und Hexylen-1,6-Reste genannt. An die Alkylenreste können weitere Ringe annelliert sein, wie dies z. B. in den vom Cholestan abgeleiteten Verbindungen der Fall ist.

Die vorstehend für $R^1$, $R^2$ und $R^6$ genannten Reste können gegebenenfalls durch Halogen, Alkyl-, Alkoxy-, Nitril-, Formyl-, Alkylcarbonyl-, Alkoxycarbonyl-, Aminocarbonyl- oder Dialkylaminocarbonyl-Gruppen substituiert sein.

Für $R^3$ und $R^4$ seien als Alkylreste beispielsweise genannt: $C_1$-$C_8$-Alkylreste wie der Methyl-, Ethyl-, n-Propyl-, iso-Propyl-, n-Butyl-, iso-Butyl-, tert.-Butyl-, n-Pentyl-, 2-Methylpentyl-, 3-Methylpentyl-, n-Hexyl-, i-Hexyl-, 2-Ethylhexyl-Rest; als Cycloalkylreste Cyclopropyl-, Cyclobutyl-, Cyclopentyl-, Cyclohexyl-, Cycloheptyl- oder Cyclooctyl-Reste; als Aralkylreste insbesondere der Benzyl- und 2-Phenylethyl-Rest; als Arylreste insbesondere der Phenyl- oder Naphthyl-Rest. Die Aralkyl- und Arylreste können durch Halogen oder niedere Alkyl- oder niedere Alkoxygruppen substituiert sein.

Die Natur der Reste $R^3$, $R^4$ und $R^6$ ist für den Verlauf der erfindungsgemäßen Invertierungsreaktion ohne Bedeutung. Aus Kostengründen verwendet man möglichst einfach und daher billig zugängliche Carbodiimide (VI) und Carbonsäuren (III); als Carbodiimide z. B. Dicyclohexylcarbodiimid oder Diisopropylcarbodiimid und als Carbonsäuren z. B. Ameisen-, Essig-, Benzoe-, Acryl- oder Methacrylsäure.

Die Addition der sekundären Alkohole der Formel (I) an die Carbodiimide der Formel (VI) kann nach bekannten Verfahren (siehe Synthesis 1979, 561; Angew. Chem. 78, (1966) 483) in der dort beschriebenen Weise vorgenommen werden, indem man die Alkohole der Formel (I) in Gegenwart von Lewis-Säuren, gegebenenfalls in einem organischen Lösungsmittel, mit den Carbodiimiden der Formel (VI) zu den Isoharnstoffethern der Formel (II) umsetzt.

Als Lewis-Säuren kommen für die Additionsreaktion beispielsweise Kupfer(I)- und Kupfer(II)halogenide, Titan(IV)halogenide, Titan(IV)alkoholate, Zink-, Zinn-, Eisen- oder Borhalogenide in Betracht; bevorzugt werden Kupfer(I)- oder -(II)-chlorid eingesetzt. Die Lewis-Säuren werden in Mengen von 0,01 bis 5 Mol-%, vorzugsweise von 0,1 bis 1 Mol-%, zugesetzt.

Die Addition der sekundären Alkohole an die Carbodiimide kann in Abwesenheit von Lösungsmitteln vorgenommen werden, doch können auch aprotische organische Lösungsmittel wie Kohlenwasserstoffe, Ether, Säureamide oder Nitrile verwendet werden. Für den Fall, daß man die Additionsreaktion in einem Lösungsmittel ausführt, wählt man für diese Reaktion zweckmäßig dasselbe Lösungsmittel, wie es in der nachfolgenden Umsetzung der Isoharnstoffether mit den Carbonsäuren verwendet wird.

Die Reaktion der sekundären Alkohole mit den Carbodiimiden wird bei Temperaturen von 0 bis 200°C, vorzugsweise 20 bis 100°C, gegebenenfalls in einer Inertgasatmosphäre durchgeführt. Bei der Umsetzung der sekundären Alkohole mit den Carbodiimiden werden Alkohol und Carbodiimid in Molverhältnissen von 1 : 1 bis 1 : 1,5, vorzugsweise 1 : 1 bis 1 : 1,1 eingesetzt. Die Isoharnstoffether (II) werden im allgemeinen nicht isoliert, sondern unmittelbar, wie sie nach der Umsetzung der sekundären Alkohole mit den Carbodiimiden anfallen, mit den Carbonsäuren umgesetzt. Grundsätzlich ist jedoch eine Isolierung der Isoharnstoffether durch Destillation oder Kristallisation möglich.

Die Isoharnstoffether der Formel (II) werden anschließend mit den Carbonsäuren (III) unter Inversion der Alkohol-Konfiguration zu den Estern (IV) umgesetzt. Diese Umsetzung wird in der Weise ausgeführt, daß man die Isoharnstoffether in einem aprotischen Lösungsmittel löst und bei Temperaturen von 0 bis 150°, vorzugsweise 10 bis 50°C mit 1 bis 1,5, vorzugsweise 1 bis 1,1 Äquivalenten Carbonsäure versetzt und das Gemisch bei Temperaturen von 20 bis 200°C, vorzugsweise der Siedetemperatur des verwendeten Lösungsmittels zur Reaktion bringt. Die Reaktionsdauer beträgt in Abhängigkeit von der Ansatzgröße und der Reaktionsfähigkeit des verwendeten Isoharnstoffethers 2 bis 24 Stunden.

Während der Reaktion fällt der aus dem Isoharnstoffether entstehende substituierte Harnstoff aus. Er kann auf einfache Weise, z. B. durch Filtrieren, entfernt werden.

Als aprotische organische Lösungsmittel werden für die Veresterungsreaktion vorzugsweise aliphatische oder aromatische Kohlenwasserstoffe wie Petrolether, Cyclohexan, Benzol oder Toluol; Ether wie Diethylether, Tetrahydrofuran oder Dioxan verwendet; Säureamide, Dimethylformamid oder Nitrile wie Acetonitril sind auch einsetzbar. Bevorzugt werden jedoch Toluol oder Dioxan verwendet. Die nach der Umsetzung vorliegenden Reaktionslösungen werden in üblicher Weise vom Lösungsmittel befreit. Die als Rückstand zurückbleibenden Ester der Formel (IV) können ohne weitere Reinigung unmittelbar verseift oder aber zunächst durch Destillation, Kristallisation oder Säulenchromatographie gereinigt und anschließend verseift werden.

Die Verseifung von Estern der Formel (IV) unter Konfigurationserhalt zu den sekundären Alkoholen (V) ist eine bekannte Reaktion der organischen Chemie. Sie wird üblicherweise durch Umsetzung des Esters (IV) mit Alkali- oder Erdalkalihydroxiden oder Alkalialkolaten durchgeführt. Bevorzugt wird die Verseifung durch Verrühren des Esters (IV) mit 0,1 bis 10 Mol-%, bevorzugt 1 bis 5 Mol-% Natriummethylat in Methanol bei Raumtemperatur vorgenommen. Der sekundäre Alkohol mit invertierter Konfiguration wird anschließend aus dem Verseifungsgemisch durch Destillation isoliert.

Besitzt der als Ausgangsverbindung verwendete sekundäre Alkohol der Formel (I) an dem die Hydroxygruppe tragenden Kohlenstoffatome die R-Konfiguration, so weist der nach dem erfindungsgemäßen Verfahren erhaltene Ester (III) und der aus diesem gewonnene sekundäre Alkohol der Formel (V) die S-Konfiguration auf und umgekehrt.

Das erfindungsgemäße Verfahren sei am Beispiel des (-)-Menthols erläutert:

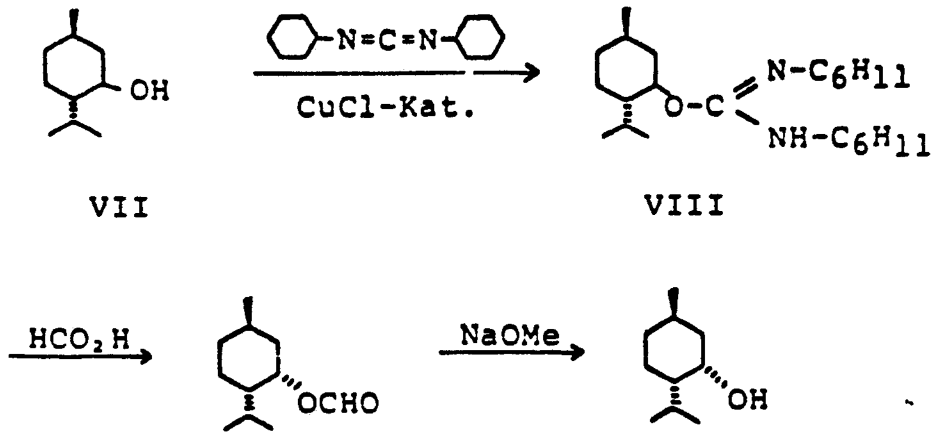

VII VIII

IX X

Optisch reines (-)-Menthol (VII) (R-Konfiguration an dem die Hydroxygruppe tragenden Kohlenstoffatom) wird in der oben erläuterten Weise an Dicyclohexylcarbodiimid addiert. Der entstandene Isoharnstoffether (VIII) wird unmittelbar, d. h, ohne Zwischenreinigung, mit Ameisensäure umgesetzt. Man erhält in hoher Ausbeute unter Inversion der Konfiguration Neomenthylformiat (IX) (S-Konfiguration an dem die Estergruppe tragenden Kohlenstoffatom; Enantiomerenüberschuß > 99 %). Durch Verseifung erhält man aus diesem Ester Neomenthol (X), das sich von (-)-Menthol (VII) durch die S-Konfiguration an dem die Hydroxygruppe tragenden Kohlenstoffatom unterscheidet (Enantiomerenüberschuß > 99 %).

Analog zum (-)-Menthol wurden die in der folgenden Tabelle 1 angegebenen sekundären Alkohole in der ebenfalls in der Tabelle angegebenen Ausbeute und mit den in der Tabelle angegebenen Enantiomerenüberschüssen invertiert.

**Tabelle 1**

| Sekundärer Alkohol (I) | Konfig. an C-OH | Carbonsäure (III) | Invertierter Ester (IV) Ausbeute[a] | e.e.[a] | Konfig. | Invertierter Alkohol (V) Ausbeute | e.e.[a] | Konfig. |
|---|---|---|---|---|---|---|---|---|
| 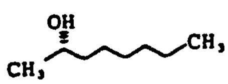 | S | $CH_3COOH$<br>HCOOH | 66 %<br>83 % | > 99 %<br>> 99 % | R<br>R | 90 % | 99 % | R |
| (±) 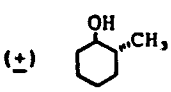 | R, S | HCOOH | 50 % | > 99 %[b] | S, R | 91 % | 99 %[b] | S, R |
| 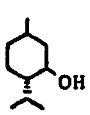 | R<br>R<br>R | HCOOH<br>$CH_2=CH-CO$<br>$CH_2=C(CH_3)-COO$ | 80 %<br>51 %<br>64 % | > 99 %<br>> 99 %<br>> 99 % | S<br>S<br>S | 90 % | 99 % | S |
| 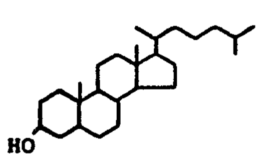 | S | HCOOH | > 75 % | > 99 % | R | 90 % | > 99 % | R |
| 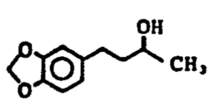<br>XI | S : R<br>79 : 21 | HCOOH | 84 % | - | - | 89 % | S : R =<br>31 : 69 | XII |

[a] Enantiomerenüberschuß
[b] Diastereomerenüberschuß

Das erfindungsgemäße Verfahren weist gegenüber den bekannten Verfahren folgende wesentliche Vorteile auf, die das Verfahren auch für eine Anwendung in technischem Maßstab interessant machen:

Das erfindungsgemäße Verfahren verläuft in hohen Ausbeuten und in ausgezeichneter Stereoselektivität; die Reaktion verläuft unter milden, annähernd neutralen Bedingungen, so daß sie auch für die Inversion der Konfiguration solcher sekundärer Alkohole anwendbar ist, die empfindliche funktionelle Gruppen enthalten; die Reaktion läßt sich in einer Stufe durchführen, da die intermediär entstehenden Isoharnstoffether nicht isoliert werden müssen, sondern unmittelbar mit den Carbonsäuren umgesetzt werden können; die Aufarbeitung des bei der Esterbildung anfallenden Reaktionsgemisches und die Abtrennung des Hilfsreagenzes (substituierten Harnstoffs) ist besonders einfach, da die neben den Estern aus den Isoharnstoffen entstehenden substituierten Harnstoffe unter den Reaktionsbedingungen ausfallen und bereits mechanisch, z. B. durch Filtration, entfernt werden können; außerdem sind die als Hilfsreagenzien erforderlichen Carbodiimide und Carbonsäuren technisch leicht zugänglich und deshalb preiswert. Das erfindungsgemäße Verfahren ist ganz allgemein von Nutzen, um schwer zugängliche Stereoisomere sekundärer Alkohole oder deren Ester aus leicht zugänglichen zu gewinnen. Derartige Verbindungen, besonders solche in enantiomerenreiner Form, sind als Zwischenprodukte, z. B. für Wirkstoffe in der Pharma oder in der Landwirtschaft, von Interesse, da sich bekanntlich Stereoisomere oft wesentlich in ihrer Wirksamkeit unterscheiden.

Es kann von Vorteil sein, ein Enantiomeres eines sekundären Alkohols durch Inversion des optischen Antipoden zu gewinnen, wenn beispielsweise nur der optische Antipode als Naturstoff zugänglich ist oder sich nur der Antipode durch mikrobiologische Reduktion in einfacher Weise gewinnen läßt. Das erfindungsgemäße Verfahren kann auch mit Erfolg angewendet werden, um im Anschluß eine Racematspaltung eines sekundären Alkohols das unerwünschte Enantiomere zurückzuführen, d. h. durch Inversion ebenfalls in das gewünschte Stereoisomere zu überführen. Ferner ist das erfindungsgemäße Verfahren von hohem Interesse, um auf einfache Weise polymerisierbare Ester, z. B. Acrylate oder Methacrylate optisch aktiver sekundärer Alkohole, in enantiomerenreiner Form zu gewinnen, die zu optisch aktiven Polymeren polymerisiert werden können.

Das erfindungsgemäße Verfahren sei anhand der folgenden Beispiele erläutert.

**Beispiel 1**

a) Veresterung unter Inversion der Konfiguration

156 g (1 Mol) (-)-Menthol ($[\alpha]^{20}_D$ = -48,5° (C = 5, EtOH)) werden mit 248 g (1,2 Mol) Dicyclohexylcarbodiimid und 100 mg Kupfer(I)-chlorid 3 Tage bei Raumtemperatur gerührt. Das Reaktionsgemisch wird in 400 ml trockenem Toluol aufgenommen, die Lösung mit 45 ml (1,2 Mol) konzentrierter Ameisensäure versetzt und 20 Stunden bei 110°C gerührt.

Der ausgefallene Dicyclohexylharnstoff wird abfiltriert und mit Methylenchlorid gewaschen. Das organische Filtrat wird im Vakuum vom Lösungsmittel befreit. Der Rückstand wird in Ether aufgenommen. Die Etherlösung wird mit Natriumbicarbonatlösung neutral gewaschen, dann über Natriumsulfat getrocknet und anschließend im Vakuum vom Lösungsmittel befreit. Der Rückstand wird destilliert.

Es werden 147 g (80 % der Theorie) Neomenthylformiat erhalten. Kp.: 52 - 60°C/0,25 mbar; $[\alpha]^{20}_D$ = +51,9° (unverdünnt); Enantiomerenüberschuß e.e. > 99 %.

b) Verseifung

Die Mischung aus 122,5 g (0,67 Mol) Neomenthylformiat, 6 ml 30-%-iger Natriummethylatlösung und 250 ml Methanol wird 12 Stunden bei Raumtemperatur gerührt. Nach dem Abdestillieren des Methanols wird der Rückstand im Vakuum destilliert.

Es werden 93,4 g (90 % der Theorie) Neomenthol erhalten (Kp.: 94 - 98°C/18 mbar; $[\alpha]^{20}_D$ = +19,6° (C = 1, EtOH); Mentholgehalt gemäß Kapillar-Gaschromatographie < 0,2 %; Enantiomerenüberschuß e.e. > 99 %.

**Beispiel 2**

61,8 g (0,4 Mol) (-)-Menthol werden mit 50 g (0,4 Mol) Diisopropylcarbodiimid und 50 mg Kupfer(I)-chlorid 3 Tage bei Raumtemperatur verrührt. Der gebildete Isoharnstoffether wird durch Destillation isoliert.

Man erhält 101 g (90 % der Theorie) O-(-)-Menthyl-N,N'-diisopropyl-isoharnstoffether; Kp.: 80 - 90°C/0,03 mbar.

Die 101 g Isoharnstoffether werden mit 16 ml Ameisensäure in 200 ml trockenem Toluol wie in Beispiel 1a) beschrieben umgesetzt.

Es werden 54,5 g (83 % der Theorie) Neomenthylformiat erhalten.

**Beispiel 3**

Der gemäß Beispiel 1a) aus 156 g (1 Mol) (-)-Menthol und 248 g (1,2 Mol) Dicyclohexylcarbodiimid hergestellte Isoharnstoffether wird mit 82 ml (1,2 Mol) Acrylsäure in 400 ml trockenem Toluol 20 Stunden bei 110°C gerührt. Das Estergemisch wird wie in Beispiel 1a) beschrieben aufgearbeitet. Durch Destillation werden 106 g (51 % der Theorie) Neomenthylacrylat erhalten; Kp.: 60 - 65°C/0,1 mbar; $[\alpha]^{20}_D$ = +47,1 (C = 1, EtOH).

Eine zum Konfigurations-Beweis gemäß Beispiel 1b) verseifte Probe des Neomenthylacrylats enthielt gemäß Kapillar-Gaschromatographie neben Neomenthol nur < 1 % Menthol (Enantiomerenüberschuß > 99 %).

Werden anstelle der 1,2 Mol Acrylsäure 102 ml (1,2 Mol) Methacrylsäure verwendet, so werden 143 g (64 % der Theorie) Neomenthyl-methacrylat erhalten; Kp.: 65 - 72°C/0,1 mbar. Die optische Reinheit des Neomenthylmethacrylats ist mit der optischen Reinheit des Neomentylacrylats vergleichbar.

**Beispiel 4**

Der nach der in Beispiel 1a) beschriebenen Weise aus 6,5 g (50 mmol) (S)-2-Octanol ($[\alpha]^{20}_D$ = +9,59° unverdünnt) und 12,4 g (60 mmol) Dicyclohexylcarbodiimid gewonnene Isoharnstoffether wird in 30 ml wasserfreiem Dioxan mit 2,76 g (60 mmol) wasserfreier Ameisensäure 20 Stunden bei 100°C gerührt. Die Reaktionsmischung wird wie in Beispiel 1a) beschrieben aufgearbeitet.

Man erhält nach der Destillation 6,55 g (83 % der Theorie) (R)-2-Formyloxy-octan; Kp.: 35°C/0,04 mbar; $[\alpha]^{20}_D$ = +3,75° (unverdünnt); Enantiomerenüberschuß e.e. > 99 %.

Die Verseifung des Esters mit 0,15 ml 30-%-iger Natriummethylatlösung in 10 ml Methanol in der in Beispiel 1b) beschriebenen Weise ergibt 4,69 g (90 % der Theorie) (R)-2-Octanol; Kp.: 50°/0,1 mbar; $[\alpha]^{20}_D$ = -9,36° (unverdünnt); Enantiomerenüberschuß e.e. > 99 %.

**Beispiel 5**

Es wird wie in Beispiel 4 beschrieben verfahren, jedoch wird anstelle der Ameisensäure 3,6 g (60 mmol) Eisessig verwendet und das Reaktionsgemisch 8 Stunden bei 100°C gerührt.

Es werden 5,71 g (66 % der Theorie) (R)-2-Acetoxy-octan erhalten; Kp.: 70 - 75°C/0,5 mbar; $[\alpha]^{20}_D$ = -6,32° (unverdünnt); Enantiomerenüberschuß e.e. > 99 %.

Die Verseifung des (R)-2-Acetoxy-octans zum (R)-2-Octanol verläuft wie in Beispiel 4 für das Formiat beschrieben.

**Beispiel 6**

Der gemäß Beispiel 1a) aus 20 g (0,175 Mol) (±)trans-2-Methyl-cyclohexanol und 43,3 g (0,21 Mol) Dicyclohexylcarbodiimid dargestellte Isoharnstoffether wird mit 7,9 ml (0,21 Mol) wasserfreier Ameisensäure und 80 ml wasserfreiem Toluol 20 Stunden bei Rückflußtemperatur umgesetzt. Das Veresterungsgemisch wird wie in Beispiel 1 beschrieben aufgearbeitet.

Es werden 12,4 g (50 % der Theorie) (±)cis-1-Formyloxy-2-methylcyclohexan erhalten; Kp.: 65 - 70°C/16 mbar.

Die Verseifung von 5 g (35 mmol) dieses (±)cis-1-Formyloxy-2-methylcyclohexans mit 0,3 ml 30-%-iger Natriummethylatlösung in 15 ml Methanol gemäß Beispiel 1b) ergibt 3,66 g (91 % der Theorie) (±)cis-2-Methylcyclohexanol; Kp.: 75°C/18 mbar; gemäß Kapillar-Gaschromatographie ist der cis-Anteil > 98 %, der trans-Anteil < 1 %; der Diastereomeren-Überschuß d.e. ist > 99 %.

**Beispiel 7**

19,4 g (50 mmol) Dihydrocholesterin werden mit 12,4 g (60 mmol) Dicyclohexylcarbodiimid und 20 mg Kupfer(I)-chlorid 3 Tage bei 60°C in 30 ml wasserfreiem Dioxan gerührt. Anschließend versetzt man das Reaktionsgemisch mit 2,80 g (60 mmol) wasserfreier Ameisensäure. Das Veresterungsgemisch wird 20 Stunden bei 100°C gerührt und anschließend wie in Beispiel 1a) beschrieben aufgearbeitet.

Es werden 20,8 g (100 % der Theorie) rohes, bereits sehr reines 3α-Formyloxy-5α-cholestan erhalten; Fp.: 107 - 108°C.

Durch säulenchromatographische Reinigung an Kieselgel (Laufmittel: Petrolether/Ether 10 : 1) werden 14,4 g (= 75 % der Theorie) reines 3α-Formyloxy-5α-cholestan erhalten; $[\alpha]^{20}_D$ = +29,5° (c = 1, $CHCl_3$); Enantiomerenüberschuß e.e. > 99 %.

Die Verseifung von 7,0 g (16,8 mmol) des Rohproduktes mit 0,1 ml 30-%-iger Natriummethylatlösung in der in Beispiel 1b) beschriebenen Weise ergibt 6,30 g (= 97 % der Theorie) 3α-Hydroxy-5α-cholestan. Die Verbindung wird durch Chromatographie an Kieselgel (Laufmittel: Methylenchlorid/Methanol 95 : 5) gereinigt. Es werden 5,84 g (= 90 % der Theorie) reines 3α-Hydroxy-5α-cholestan; Fp.: 177 - 179°C erhalten.

**Beispiel 8**

Der gemäß Beispiel 1a) aus 9,7 g (50 mmol) des Alkohols XI (Enantiomerenverhältnis S : R = 79 : 21) und 11,4 g (55 mmol) Dicyclohexylcarbodiimid hergestellte Isoharnstoffether wird mit 2,3 g (50 mmol) wasserfreier Ameisensäure in 20 ml wasserfreiem Toluol 20 Stunden bei 100°C gerührt. Das Veresterungsgemisch wird wie in Beispiel 1a) beschrieben aufgearbeitet.

Nach der Destillation erhält man 9,34 g (= 84 % der Theorie) des invertierten Formiats.

Die Verseifung von 8,80 g (40 mmol) dieses Formiats mit 0,4 ml 30-%-iger Natriummethylatlösung in 20 ml Methanol gemäß Beispiel 1b) und Destillation liefert 6,85 g (= 89 % der Theorie) des invertierten Alkohols XII; Kp.: 125°C/0,1 mbar (Enantiomerenverhältnis S : R = 31 : 69).

**Patentansprüche**

1. Verfahren zur Inversion der Konfiguration sekundärer Alkohole, bei denen die Hydroxygruppe an ein asymmetrisches Kohlenstoffatom geknüpft ist, durch Veresterung dieser sekundären Alkohole unter Inversion ihrer Konfiguration und nachfolgendes Verseifen der Ester unter Erhalt der Konfiguration der sekundären Alkohole, dadurch gekennzeichnet, daß man die Veresterung unter Inversion der Konfiguration in der Weise vornimmt, daß man den sekundären Alkohol durch Addition an ein Carbodiimid zunäschst in einen Isoharnstoffether überführt und diesen Isoharnstoffether mit einer Carbonsäure zum Ester der Carbonsäure umsetzt.

2. Verfahren gemäß Anspruch 1, dadurch gekennzeichnet, daß man die Umsetzung des Isoharnstoffethers mit der Carbonsäure in einem aprotischen Lösungsmittel vornimmt.

3. Verfahren gemäß Anspruch 1, dadurch gekennzeichnet, daß man sekundäre Alkohole der Formel

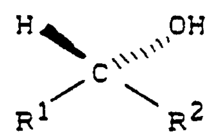

in der

$R^1$ und $R^2$ unabhängig voneinander für einen gegebenenfalls substituierten gesättigten oder ungesättigten aliphatischen oder cycloaliphatischen Kohlenwasserstoffrest oder einen gegebenenfalls substituierten araliphatischen oder aromatischen Kohlenwasserstoffrest stehen oder zusammen einen asymmetrischen Alkylenrest bilden, mit der Maßgabe, daß $R^1 \neq R^2$ ist,

an Carbodiimide der Formel

$R^3\text{-}N=C=N\text{-}R^4$,

in der

$R^3$ und $R^4$ unabhängig voneinander für einen Alkyl-, Cycloalkyl-, Aralkyl- oder Arylrest stehen

addiert und die dabei gebildeten Isoharnstoffether der Formel

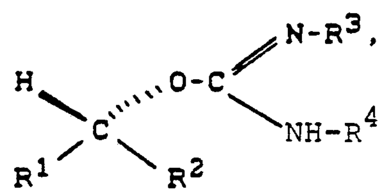

in der

$R^1$, $R^2$, $R^3$ und $R^4$ die vorstehend angegebene Bedeutung haben,

mit Carbonsäuren der Formel

$R^6\text{-}CO_2H$,

in der

$R^6$ für Wasserstoff oder einen gegebenenfalls substituierten gesättigten oder ungesättigten aliphatischen oder cycloaliphatischen Kohlenwasserstoffrest oder einen gegebenenfalls substituierten aromatischen Kohlenwasserstoffrest steht,

unter Inversion der Alkohol-Konfiguration zu Estern der Formel

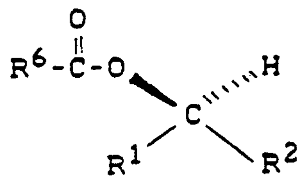

,

umsetzt, in der

$R^1$, $R^2$ und $R^6$ die vorstehend genannte Bedeutung haben.

## Claims

1. Process for inverting the configuration of secondary alcohols in which the hydroxyl group is attached to an asymmetric carbon atom, by esterification of these secondary alcohols, with inversion of their configurations and subsequent saponification of the esters, with retention of the configuration of the secondary alcohols, characterized in that the esterification with inversion of the configuration is carried out by first converting the secondary alcohol into an isourea ether by an addition reaction with a carbodiimide and reacting this isourea ether with a carboxylic acid to give the ester of the carboxylic acid.

2. Process according to Claim 1, characterized in that the reaction of the isourea ether with the carboxylic acid is carried out in an aprotic solvent.

3. Process according to Claim 1, characterized in that secondary alcohols of the formula

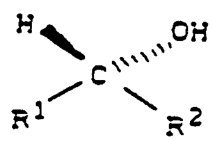

in which

$R^1$ and $R^2$ independently of one another represent an optionally substituted, saturated or unsaturated, aliphatic or cycloaliphatic hydrocarbon radical or an optionally substituted araliphatic or aromatic hydrocarbon radical, or together form an asymmetric alkylene radical, subject to the proviso that $R^1$ is not identical with $R^2$,

are subjected to an addition reaction with carbodiimides of the formula

$R^3\text{-N}=\text{C}=\text{N-}R^4$

in which

$R^3$ and $R^4$ independently of one another represent an alkyl, cycloalkyl, aralkyl or aryl radical,

and the resulting isourea ethers of the formula

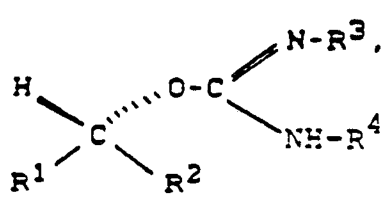

in which

$R^1$, $R^2$, $R^3$ and $R^4$ have the meaning indicated above,

are reacted with carboxylic acids of the formula

$R^6\text{-}CO_2H$

in which

$R^6$ represents hydrogen or an optionally substituted, saturated or unsaturated, aliphatic or cycloaliphatic

hydrocarbon radical or an optionally substituted aromatic hydrocarbon radical, with inversion of the configuration of the alcohol, to give esters of the formula

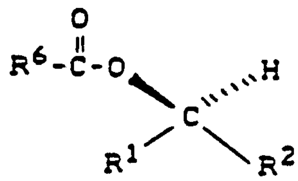

in which

$R^1$, $R^2$ and $R^6$ have the meaning mentioned above.

**Revendications**

1. Procédé pour l'inversion de la configuration d'alcools secondaires dans lesquels les groupes hydroxyles sont liés à un atome de carbone asymétrique par estérification de ces alcools secondaires avec inversion de leur configuration et saponification ultérieure des esters, en conservant la configuration des alcools secondaires, caractérisé en ce que l'on effectue l'estérification avec inversion de la configuration en transformant tout d'abord l'alcool secondaire par addition à une carbodiimide en un éther d'iso-urée et en transformant ensuite cet éther d'iso-urée par un acide carboxylique en ester de l'acide carboxylique.

2. Procédé selon la revendication 1, caractérisé en ce que la réaction de l'éther d'iso-urée avec l'acide carboxylique est effectuée dans un solvant aprotique.

3. Procédé selon la revendication 1, caractérisé en ce que l'on fait réagir les alcools de la formule

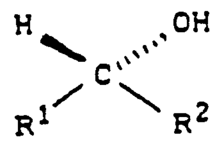

dans laquelle

$R^1$ et $R^2$ représentent, indépendamment l'un de l'autre, un radical d'hydrocarbure cycloaliphatique ou aliphatique saturé ou insaturé, éventuellement substitué, ou un radical d'hydrocarbure aromatique ou araliphatique, éventuellement substitué, ou forment ensemble un radical alkylénique asymétrique avec la condition que $R^1 \neq R^2$,

avec des carbodiimides de la formule

$R^3\text{-}N=C=N\text{-}R^4$

dans laquelle

$R^3$ et $R^4$ représentent, indépendamment l'un de l'autre, un radical alkyle, cycloalkyle, aralkyle ou aryle tandis que l'éther d'iso-urée ainsi obtenu et répondant à la formule

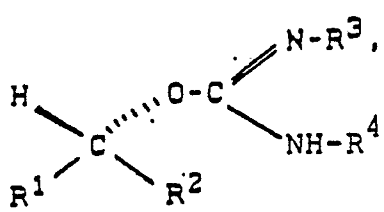

dans laquelle

$R^1$, $R^2$, $R^3$ et $R^4$ ont les significations précédentes,

est mis en réaction avec des acides carboxyliques de la formule

$R^6\text{-}CO_2H$,

dans laquelle

$R^6$ représente l'hydrogène ou un radical d'hydrocarbure cycloaliphatique ou aliphatique saturé ou insaturé, éventuellement substitué ou un radical d'hydrocarbure aromatique éventuellement substitué, avec inversion de la configuration de l'alcool pour obtenir des esters répondant à la formule

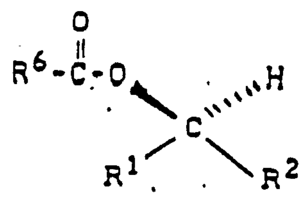

dans laquelle
$R^1$, $R^2$ et $R^6$ ont les significations précitées.